# EUROPÄISCHE PATENTSCHRIFT

(11) **EP 0 344 578 B1**
(45) Veröffentlichungstag und Bekanntmachung des Hinweises auf die Patenterteilung: **10.08.1994**
(21) Anmeldenummer: 89109247.0
(22) Anmeldetag: 23.05.1989
(51) Int. Cl.: G01N 33/531, G01N 33/543, G01N 33/53

(54) **Verfahren zur Bestimmung einer immunologisch nachweisbaren Substanz und dazu geeignetes Reaktionsgefäss**
Method to determine an immunologically detectable substance, and suitable reaction vial for this
Méthode pour déterminer une substance immunologique décelable et récipient à réaction à cet effet

(30) Priorität: 25.05.1988 DE 3817716; 20.01.1989 DE 3901638
(43) Veröffentlichungstag der Anmeldung: 06.12.1989
(73) Patentinhaber: BOEHRINGER MANNHEIM GMBH, 68298 Mannheim (DE)
(72) Erfinder: Schmitt, Urban, D-8125 Oberhausen (DE); Maurer, Eberhard, Dr., D-8120 Weilheim (DE); Rüdinger, Wolfgang, Dr.med.,Dr.Ing., D-6943 Birkenau (DE); Deeg, Rolf, Dr., D-8139 Bernried (DE)
(74) Vertreter: Huber, Bernhard, Dipl.-Chem.

(56) Entgegenhaltungen:
- DE-A- 3 640 412
- FR-A- 2 601 455
- GB-A- 2 181 840
- CHEMICAL ABSTRACTS, Band 107, 1987, Columbus, OH (US); Seite 363, Nr. 172014b#

## Beschreibung

Die Erfindung betrifft ein Verfahren zur Bestimmung einer immunologisch nachweisbaren Substanz nach dem Prinzip des heterogenen Immunoassays unter Verwendung einer Festphase, an die eine der immunologisch aktiven Reaktionskomponenten gebunden ist sowie ein dazu geeignetes Reaktionsgefäß.

Zur Bestimmung einer spezifisch bindefähigen Substanz bedient man sich häufig Verfahren nach dem Prinzip des Immunoassays. Dabei wird einer der Partner eines spezifisch miteinander bindefähigen Substanzpaares mit dem für ihn spezifischen Rezeptor, der in an sich bekannter Weise markiert ist, umgesetzt. Das Konjugat aus diesen beiden Substanzen kann dann noch mit einem Rezeptor, der für das Konjugat oder eines der beiden Teile des Konjugats spezifisch ist, umgesetzt werden. Für diese immunologischen Verfahren gibt es viele Variationen. Vorteilhaft ist es dabei, wenn einer der Rezeptoren an eine Festphase gebunden vorliegt. Dies erleichtert die Trennung von gebunden und nicht gebunden vorliegenden Reaktionspartnern. Zur Bestimmung der spezifisch bindefähigen Substanz wird dann die Menge an an der Festphase gebundenem markiertem Reaktionspartner oder an in der Lösung vorliegendem markiertem Reaktionspartner gemessen und zu der Menge an zu bestimmendem Reaktionspartner in an sich bekannter Weise in Beziehung gesetzt.

Als Festphase werden bei den immunologischen Verfahren üblicherweise Kunststoffröhrchen oder Mikrotiterplatten, an deren Innenoberfläche der Reaktionspartner fixiert ist, oder Kugeln, an deren Außenoberfläche der Reaktionspartner fixiert ist, verwendet.

Dabei muß die Bindung des spezifischen Reaktionspartners an die jeweilige Oberfläche so erfolgen, daß er nicht die Fähigkeit zur spezifischen Bindung der für ihn spezifisch bindefähigen Substanz verliert. Aus diesem Grunde erfolgt die Bindung des Reaktionspartners an die Festphase meistens adsorptiv. Es wurde daher schon vorgeschlagen, die Fixierung des Reaktionspartners an die Festphase über ein Kupplungsmittel, das die Bindung vermittelt, zu bewirken. Dabei muß auch wieder darauf geachtet werden, daß die Bindung des Reaktionspartners an das Bindungsmittel nicht die spezifisch reagierende Region des Moleküls zerstört bzw. daß der Reaktionspartner so gebunden wird, daß seine reaktive Stelle von der Festphase weg dem Bindungspartner zugewandt ist.

Aus der FR-A 2601455 ist es bekannt, eine Mischung aus Avidin, Biotin und einer reaktiven Substanz auf einer Festphase abzuscheiden, so daß man im Ergebnis eine mit der reaktiven Substanz beschichtete Oberfläche erhält. Eine derartige Oberfläche ist nur zur Bindung des speziellen Analyten geeignet, der mit der reaktiven Substanz bindefähig ist.

Nachteil aller dieser bekannten Verfahren ist es, daß für jede spezifische Reaktion eine spezielle Festphase zur Verfügung gestellt werden muß. Das bedeutet, daß für jeden einzelnen Test eine andere Festphase hergestellt, gelagert und dann zur Bestimmung eingesetzt werden muß, was aufwendig ist.

Es war daher Aufgabe der Erfindung, ein Verfahren und ein Reaktionsgefäß zur Verfügung zu stellen, das für viele verschiedene Nachweisverfahren geeignet ist. In der klinischen Routinediagnostik werden aus einer Blutprobe jeweils viele verschiedene Parameter wie Hormone, Tumormarker, Infektions-, Allergie- und Fertilitätsparameter bestimmt. Die Konzentration der zu bestimmenden Parameter umfaßt einen weiten Bereich. Niedrig konzentrierte Parameter wie z. B. TSH und Prolactin liegen im Bereich von 10⁻¹⁰ bis 10⁻¹² mol/l, während hoch konzentrierte Parameter wie T₃ und T₄ bei ca. 10⁻⁷ bis 10⁻⁸ mol/l liegen. Dabei wäre es wünschenswert, wenn für die einzelnen Bestimmungen dabei nicht jedesmal verschiedene Röhrchen verwendet werden müßten, sondern wenn ein Einheits-Reaktionsgefäß für alle Bestimmungen eingesetzt werden könnte.

Diese Aufgabe wird gelöst durch ein Verfahren zur Bestimmung einer immunologisch nachweisbaren Substanz nach dem Prinzip des heterogenen Immunoassays unter Verwendung einer Festphase, an die eine der immunologisch aktiven Reaktionskomponenten gebunden ist, das dadurch gekennzeichnet ist, daß man als feste Phase ein Reaktionsgefäß verwendet, an dessen Innenoberfläche Streptavidin oder Avidin in einer solchen Menge gebunden ist, daß pro ml Reaktionsvolumen 0,1 bis 2,5 µg vorhanden sind. Diese Mengenangabe bezieht sich auf Streptavidin oder Avidin, welches für den Bindepartner im Rahmen der immunologischen Reaktion, der in Form eines biotinylierten Proteins oder Haptens vorliegt, zugänglich ist.

Das erfindungsgemäße Verfahren ist anwendbar für alle Bestimmungsverfahren, bei denen der Rezeptor, der z. B. ein Antikörper oder ein Antigen sein kann, der an der Festphase immobilisiert werden soll, mit Biotin konjugiert ist. Das erfindungsgemäß festphasenfixierte Streptavidin bzw. Avidin weist auf allen Tubematerialien eine sehr gute Haftung auf und die Bindung bleibt auch gegenüber Detergentien stabil. Bei der Erstellung von Eichkurven, die zur Auswertung bei vielen immunologischen Verfahren notwendig sind, liefert das erfindungsgemäß festphasengebundene Streptavidin bzw. Avidin steile Eichkurven bei geringen Leerwerten, was zu einer Erhöhung der Genauigkeit führt. Ein weiterer Vorteil ist, daß Chargenschwankungen der beschichteten Streptavidinmenge nur vernachlässigbare Einflüsse auf die Meßergebnisse hatten. Der besondere Vorteil des erfindungsgemäßen Verfahrens liegt darin, daß die gebundene Menge an Streptavidin bzw. Avidin so eingestellt wird, daß die feste Phase für alle bekannten Verfahren nach dem Prinzip des heterogenen Immunoassays Verwendung finden kann. Außerdem besteht ein Vorteil darin, daß man mit dem erfindungsgemäßen Verfahren simultan mehrere Substanzen, insbesondere Antikörper, aber auch Antigene bestimmen kann. Dies wird durch Beispiel 7 näher erläutert.

In einer besonders bevorzugten Ausführungsform wird das als feste Phase verwendete Reaktionsgefäß gleichzeitig auch als Küvette zur photometrischen Bestimmung der Markierung verwendet. Dies ist besonders vorteilhaft, da nur ein einziges Gefäß zur Durchführung der gesamten Reaktion erforderlich ist. Das Reaktionsgefäß ist in diesem Falle so ausgestaltet, daß es zwei einander gegenüberliegende optisch durchlässige Wandbereiche aufweist. Dabei kann der gesamte Wandbereich mit Streptavidin oder Avidin beschichtet sein.

Erfindungsgemäß wird als feste Phase ein Reaktionsgefäß verwendet, an dessen Innenoberfläche Streptavidin oder Avidin gebunden ist. Die Bindung des Streptavidins oder Avidins erfolgt dabei nach den dem Fachmann bekannten Methoden (z. B. EP-A 0 122 209). Das Streptavidin oder Avidin kann entweder direkt an die feste Phase gebunden werden oder aber über Spacer oder die Bindung vermittelnde Substanzen gebunden werden.

In einer bevorzugten Ausführungsform des erfindungsgemäßen Verfahrens wird Streptavidin oder Avidin an ein lösliches Protein mit einem Molekulargewicht über etwa 500.000 gekuppelt und dann dieses Konjugat an eine hydrophobe Festphase adsorbiert, wie es z.B. in der Patentanmeldung DE-A 36 40 412 beschrieben ist. Bevorzugt wird ein Protein verwendet, das hydrophobisiert ist.

Die Hydrophobisierung kann beispielsweise durch Anwendung von Hitze, Behandlung mit Säuren, denaturierenden Agentien und/oder chaotropen Ionen und/oder durch chemische Kupplung mit einer hydrophoben Verbindung erfolgen. Die Behandlung mit diesen Agentien führt auch zu einer Erhöhung des Molekulargewichts. Die Erhöhung des Molekulargewichts kann auch erreicht werden durch Vernetzung mit einem bi- oder polyfunktionellen Proteinreagenz.

Als Säuren werden beispielsweise Essigsäure, Propionsäure, Milchsäure oder Salzsäure angewendet. Übliche Konzentrationen sind 1 bis 100 mmol/l bei Einwirkzeiten von 10 min bis 16 Stunden.

Geeignete chaotrope Ionen sind beispielsweise Thiocyanate, Jodide, Fluoride, Bromide, Perchlorate und Sulfate. Geeignete denaturierende Agentien sind beispielsweise Guanidinhydrochlorid oder Harnstoff. Üblicherweise werden hier Konzentrationen von 10 mmol/l bis 6 mol/l angewendet.

Zur Derivatisierung mit hydrophoben Verbindungen werden vorzugsweise lösliche Fettsäuren, Lipoide in nieder-oder hochmolekularer Form sowie synthetische Polymere, wie Polypropylenglykol oder lösliche Copolymere von Polystyrol, eingesetzt. Die Derivatisierung erfolgt nach den dem Fachmann geläufigen Methoden.

Die Vernetzung über bi- oder polyfunktionelle Verbindungen wird mit an sich bekannten Proteinbindungsreagenzien durchgeführt. Dies sind Verbindungen, die mindestens zwei funktionelle Gruppen tragen, die gleich oder verschieden sein können und die über diese funktionellen Gruppen mit funktionellen Gruppen von Proteinen reagieren können. Bevorzugt werden Verbindungen verwendet, die aus einer Alkylkette bestehen, an deren Enden sich Succinimid-, Maleimid- und/oder Aldehydgruppen befinden.

Das Protein wird mit der bi- oder polyfunktionellen Verbindung in an sich bekannter Weise vernetzt, indem das lösliche Protein und die bi- oder polyfunktionelle Verbindung umgesetzt werden.

Bevorzugt werden zur Hydrophobisierung und/oder Vernetzung Proteine mit einem Molekulargewicht von 10.000 bis 700.000 verwendet. Besonders bevorzugt wird Rinderserumalbumin, Lipase oder Immun-γ-Globulin.

An das Protein wird dann in an sich bekannter Weise das Streptavidin oder Avidin gekuppelt. Geeignete Kupplungsmethoden sind beispielsweise in Ishikawa, J., Immunoassay 4 (1983), 209-327, beschrieben.

Das erhaltene Konjugat aus Streptavidin oder Avidin und Protein wird dann adsorptiv an der als Festphase dienenden Kunststoffoberfläche adsorbiert. Bevor das Konjugat aus Protein und Streptavidin oder Avidin an die hydrophobe Festphase adsorbiert wird, ist es auch möglich, die Festphase physikalisch oder chemisch vorzubehandeln. So kann beispielsweise eine Kunststoffoberfläche vorgequollen werden oder in anderer an sich bekannter Weise aktiviert werden. Die adsorptive Bindung an die Festphase erfolgt über starke und schwache Wechselwirkungen, hydrophobe Kräfte, Dipol-Dipol- oder Ionen-Dipol-Interaktionen.

Als hydrophobe Festphase besonders geeignet sind Reaktionsgefäße aus Trägermaterialien mit einer Oberflächenspannung, die kleiner als die Oberflächenspannung des hydrophoben löslichen Proteins ist, d.h. hydrophober sind als Protein. Bevorzugt werden Trägermaterialien mit einer Oberflächenspannung 40 erg/cm² eingesetzt. Besonders geeignet sind Polystyrol, Polymethacrylat, Teflon, Polyamid, Copolymere aus Styrol und Acrylnitril, Glas und Celluloseprodukte.

Das erfindungsgemäß hergestellte Festphasenmaterial wird zur Bestimmung von vielen verschiedenen Parametern verwendet. Dabei wird dann eine der mit dem zu bestimmenden Parameter bindefähigen Substanzen mit Biotin konjugiert, das wiederum an das an der Festphase fixierte Streptavdidin bzw. Avidin bindet. Auf diese Weise können die aufgrund des Immunoassayverfahrens gebildeten spezifischen Komplexe immobilisiert und dann bestimmt werden. Erfindungsgemäß wird deshalb an der Festphase so viel Streptavidin bzw. Avidin gebunden, daß pro ml Reaktionsvolumen 0,1 bis 2,5 µg für die Bindung der biotinylierten, spezifisch bindefähigen Substanz zur Verfügung stehen.

Als Reaktionsvolumen wird dabei die Summe aus Probevolumen und Testreagenzvolumen bezeichnet.

Bevorzugt wird das Festphasenmaterial so beschichtet, daß 1 bis 2 µg Streptavidin bzw. Avidin für die Bindung mit biotinylierter, spezifisch bindefähiger Substanz pro ml Testvolumen vorhanden sind. Die Menge der biotinylierten spezifisch bindefähigen Substanz liegt beim Verfahren der Erfindung vorzugsweise im Bereich von 10⁻¹⁶ bis 10⁻⁸ mol/Reaktionsvolumen.

Ein weiterer Gegenstand der Erfindung ist ein Reaktionsgefäß mit einander gegenüberliegenden optisch durchlässigen Wandbereichen und im übrigen, zumindest teilweise, im Bereich der zur Flüssigkeitsaufnahme vorgesehenen Innenwand mit Streptavidin oder Avidin beschichteten Wänden, wobei der für die Flüssigkeitsaufnahme bestimmte Innenraum des Behälters und der Streptavidin- bzw. Avidingehalt der Beschichtung so aufeinander abgestimmt sind, daß pro ml Reaktionsvolumen 0,1 bis 2,5 µg Streptavidin oder Avidin vorhanden sind.

Dieses Reaktionsgefäß ist besonders geeignet zur Durchführung des erfindungsgemäßen Verfahrens. Da es optisch durchlässige, einander gegenüberliegende Wandbereiche hat und außerdem eine Beschichtung mit Streptavidin bzw. Avidin aufweist, kann es gleichzeitig zur Durchführung der Reaktion und zur photometrischen Bestimmung verwendet werden. Durch die Beschichtung mit 0,1 bis 2,5 µg Streptavidin bzw. Avidin pro ml Reaktionsvolumen kann dieses Reaktionsgefäß zur Bestimmung der verschiedensten Parameter nach dem Prinzip des heterogenen Immunoassays eingesetzt werden.

Das Reaktionsgefäß stellt die Festphase dar. Es besteht aus den üblicherweise verwendeten Materialien wie Kunststoffen, Glas etc. Die optisch durchlässigen Wandbereiche bestehen aus ebenfalls bekannten Materialien. Insbesondere bevorzugt sind hier Polystyrol, Copolymere des Polystyrols, Polycarbonate, Polyacrylate und Polymethacrylate.

Die Beschichtung mit Streptavidin oder Avidin kann entweder direkt oder über ein Trägermaterial oder einen Spacer erfolgen. Geeignet ist beispielsweise die Bindung von Streptavidin oder Avidin an ein lösliches Protein mit einem Molekulargewicht über 500.000, das dann an der Innenoberfläche des Reaktionsgefäßes adsorbiert wird.

Gegenstand der Erfindung ist auch die Verwendung eines Einheits-Reaktionsgefäßes, das aus einem Gefäß, an dessen Innenoberfläche Streptavidin oder Avidin gebunden ist, in einer solchen Menge, daß für die Bestimmungsreaktion pro ml Reaktionsvolumen 0,1 bis 2,5 µg Streptavidin oder Avidin zur Verfügung stehen, besteht, zur Bestimmung verschiedener Parameter in Verfahren nach dem Prinzip des Immunoassays.

Erfindungsgemäß wird ein Verfahren und ein Reaktionsgefäß zur Verfügung gestellt, bei dem Streptavidin oder Avidin mit guter Haftung und dauerhaft an die Innenoberfläche eines Reaktionsgefäßes als Festphase fixiert wird. Die Haftung ist dabei so gut, daß auch ein Zusatz von Detergenzien nicht zu einer Ablösung der Substanz führt. Das erfindungsgemäß zur Verfügung gestellte Reaktionsgefäß ist universell einsetzbar und eignet sich zur Durchführung von Bestimmungsverfahren für sehr viele Parameter und erleichtert daher die Durchführung von Routinebestimmungen.

Die Erfindung wird im folgenden durch Figuren und Beispiele erläutert. In der Zeichnung stellen dar:
- Fig. 1: eine Eichkurvenschar für eine TSH-Bestimmung unter Verwendung von immobilisiertem Streptavidin in unterschiedlichen Mengen und biotinyliertem Anti-TSH-Antikörper;
- Fig. 2: eine Eichkurve für einen Prolactintest;
- Fig. 3: eine Eichkurve für einen CEA-Test und
- Fig. 4: eine Eichkurve für einen T4-Test.
- Fig. 5: eine Eichkurvenschar für eine AntiHBs-Bestimmung unter Verwendung von immobilisiertem Streptavidin in unterschiedlichen Mengen.

Die in den Beispielen verwendeten monoklonalen Antikörper gegen TSH stammen aus Hybridoma-Zellinien, die bei der European Collection of Animal Cell Cultures, Porton Down, GB, unter den Bezeichnungen ECACC 87 122201 und ECACC 87122202 hinterlegt wurden.

### Beispiel 1

Es wurde die Festphasenhaftung des erfindungsgemäßen Konjugats und hydrophobem Protein und Streptavidin untersucht:
- Thermisch aggregiertes RSA, im folgenden als Thermo-RSA bezeichnet, wurde in folgender Weise hergestellt: 1 g RSA wurde in 100 ml 50 mmol Kaliumphosphatlösung bei einem pH von 7,0 gelöst, auf 70 °C erhitzt und 4 Stunden unter leichtem Rühren bei dieser Temperatur gehalten. Die Lösung wurde abgekühlt, filtriert und auf eine Konzentration von 50 mg/ml eingestellt. Anschließend wurde gegen das 30fache Volumen bidest. Wasser dialysiert.
- Herstellung eines Konjugats von Streptavidin mit Thermo-RSA: Aus Streptomyces avidinii gewonnenes Streptavidin wurde mit Maleimido-hexanoyl-N-hydroxy-succinimid umgesetzt, und man erhielt auf diese Weise ein Maleimidogruppen tragendes Streptavidin. Thermo-RSA wurde mit S-Acetylmercaptobernsteinsäureanhydrid umgesetzt und anschließend die geschützten SH-Gruppen durch Zugabe von Hydroxylamin freigesetzt. Das Maleimidogruppen enthaltende Streptavidin wurde sodann mit dem SH-Gruppen enthaltenden Thermo-RSA vermischt unter Bildung des gewünschten Konjugats.
- Kunststofftubes aus Polystyrol wurden dann mit dem Streptavidin-Thermo-RSA-Konjugat beladen. Das Beladen der Tubes erfolgte mit 1,5 ml einer Streptavidin-Thermo-RSA-Lösung, wobei das molare Verhältnis der beiden Komponenten 1,8:l (10 µg/ml) in 40 mmol Natriumphosphatpuffer pH 7,4 bei 20 °C während 18 bis 24 Stunden betrug.

Nach Aussaugen der Tubes wurde 30 min bei 20 °C mit 1,8 ml einer Lösung von 2% Saccharose, 0,9 % Natriumchlorid und 0,3 % RSA nachbeladen. Nach Trocknung (24 Stunden bei 20 °C und 40 % relativer Feuchte) waren die Tubes einsatzbereit und haltbar.

### Beispiel 2

### TSH-Ein-Schritt-Test.

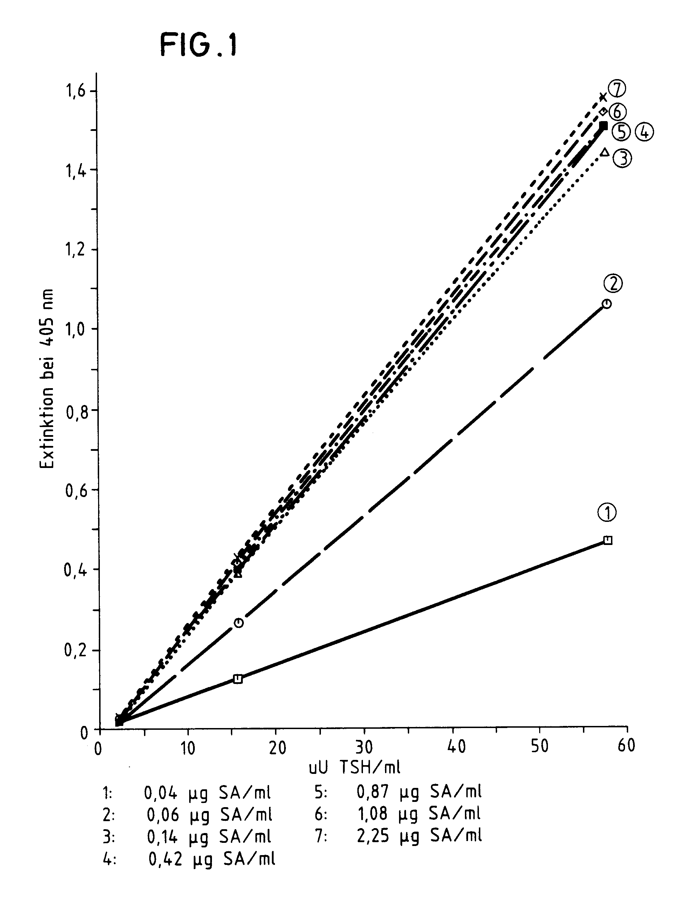

Es wurde biotinylierter monoklonaler Anti-TSH-Antikörper hergestellt. Dazu wurde der Anti-TSH-Antikörper, ECACC 87122201 , in üblicher Weise mit Biotin umgesetzt, wobei die Kopplung über die Aminogruppen erfolgt. Antikörper und Biotin wurden im Verhältnis 1:16 eingesetzt. 400 ng des so erhaltenen biotinylierten Antikörpers wurden in 1 ml Puffer gelöst (40 mmol Natriumphosphat, 0,5 % Pluronic F68, 0,2 mol Natriumtartrat/l sowie 0,01 % Phenol) und dann zusammen mit 200 µl Probe bzw. einer Standardlösung, die eine bekannte Menge an TSH enthielt, und 75 mU eines mit Peroxidase konjugierten Anti-TSH-Antikörpers (ECACC 87122202) in einem gemäß Beispiel 1 beschichteten Tube 2 Stunden inkubiert. Danach wurde dreimal mit Leitungswasser gewaschen und anschließend 1 ml ABTS-Substratlösung zugegeben. Nach einer Stunde wurde die Extinktion bei 405 nm gemessen. Die Ergebnisse sind der Fig. 1 zu entnehmen. Es zeigt sich, daß ein befriedigendes Ergebnis mit immobilisiertem Streptavidin bei einer Menge von über 0,1 µg/ml Testvolumen erhalten wird.

Es wurde der Bedarf an Streptavidin zur Bindung des in dem Test eingesetzten biotinylierten Antikörpers ermittelt. Dazu wurde zuerst die Menge an frei zugänglichem Biotin nach der Methode von Bayer, Edward A., Meir-Wilchek, Analytical Biochemistry 154 (1986), 367-370, ermittelt. Ausgehend von 1 µg Antikörper, der im Verhältnis Antikörper zu Biotin 1:15 biotinyliert wurde (Guesdon, I.L., J. Histochem. Cytochem 27 (1979) 1131-1139), ergibt sich, daß 1 bis 2 mol Biotin pro mol Antikörper zur Bindung zur Verfügung stehen, d. h. pro µg Antikörper sind 1,5 bis 3 ng Biotin frei zugänglich.

Ein mit 10 µg/ml Streptavidin-Thermo-RSA-Konjugat beladener Tube (Beispiel 1) hat eine Bindekapazität für Biotin von 15 bis 20 ng pro ml Testvolumen, bestimmt mit radioaktivem C¹⁴-Biotin.

Für biotinylierte Immun-γ-Globuline beträgt die Bindekapazität des Streptavidintubes 1,7 bis 2 µg/ml Testvolumen. Die Bestimmung wurde durchgeführt mit I¹²⁵-markiertem γ-Globulin (markiert nach der Methode von McConahey and Dixon 1966, Int. Arch. Allergy 29/185).

### Beispiel 3

### Prolactin-Test.

Prolactin wird in einem 1-Schritt-Sandwich-Immunoassay bestimmt. Zum Nachweis wird ein Reagenz mit der folgenden Zusammensetzung verwendet:
- 50 mU/ml eines Konjugats aus POD und einem Prolactinspezifischen monoklonalen Antikörper,
- 40 mmol/l Phosphatpuffer pH: 7,0
   0,5 % Pluronic F65 (Polyoxyethylenpolypropylen)
   0,2 mol/l Natriumtartrat
   0,01 % Phenol
   0,2 % Rinderserumalbumin
   400 ng/ml eines biotinylierten monoklonalen Antikörpers gegen Prolactin.

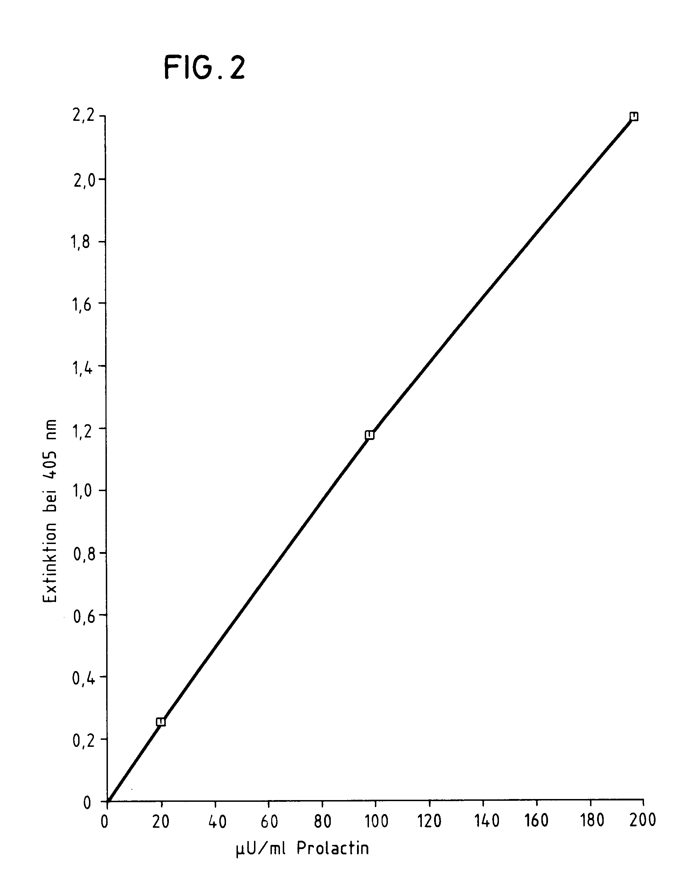

An die beiden monoklonalen Antikörper werden lediglich die Anforderungen gestellt, daß sie Prolactin erkennen und gegen verschiedene Epitope von Prolactin gerichtet sind. In einem mit Streptavidin-Thermo-RSA-Konjugat beschichteten Polystyroltube, wie es gemäß Beispiel 1 erhalten wurde, wurden 1 ml dieses Reagenzes und 50 µl Probe 30 min bei Raumtemperatur inkubiert. Anschließend wurde dreimal mit Leitungswasser gewaschen. Zur Nachweisreakion wurde 1 ml ABTS®-Substratlösung zugegeben. Nach 30 min wurde die Extinktion bei 405 nm photometrisch gemessen. Die Ergebnisse sind der Fig. 2 zu entnehmen.

ABTS®: 2,2'-Azino-di-[3-ethylbenzthiazolinsulfonat(s)]-diammoniumsalz

### Beispiel 4

### CEA-Test.

In einer Probelösung wurde CEA in einem 1-Schritt-Sandwich-Immunoassay bestimmt. Das verwendete Reagenz hatte die folgende Zusammensetzung:
- 120 mU/ml eines Konjugats aus POD und einem monoklonalen Antikörper gegen CEA,
- 40 mmol/l Phosphatpuffer pH: 7,0
   0,5 % Pluronic F68
   0,2 mol/l Natriumtartrat
   0,01 % Phenol
   0,2 % Rinderserumalbumin
   500 ng/ml eines biotinylierten monoklonalen Antikörpers gegen CEA.

An die beiden monoklonalen Antikörper gegen CEA werden lediglich die Anforderungen gestellt, daß sie CEA erkennen müssen und jeweils gegen ein anderes Epitop von CEA gerichtet sind.

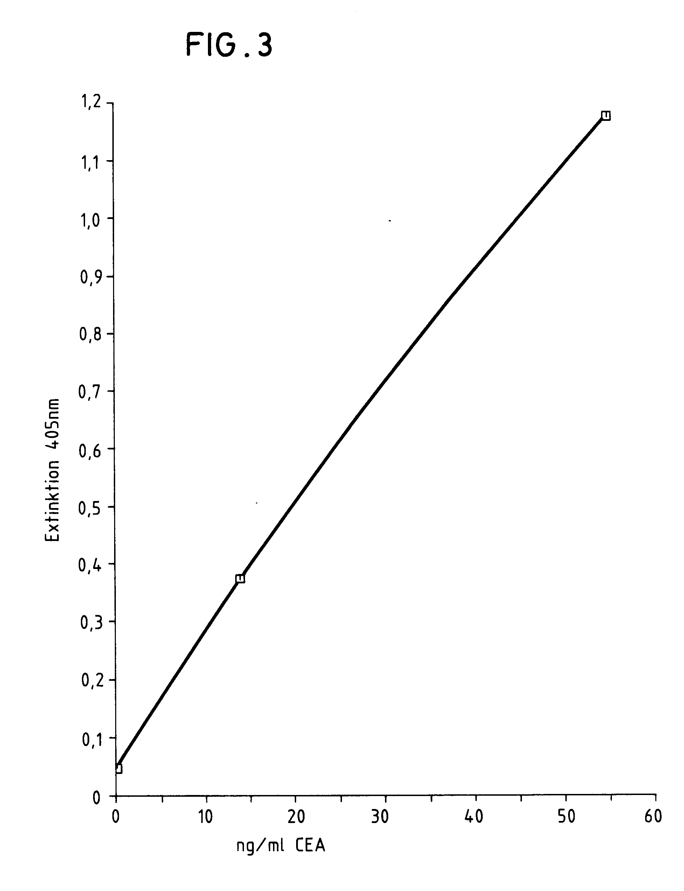

In einem mit Streptavidin-Thermo-RSA-beschichteten Polystyroltube, wie es gemäß Beispiel 1 erhalten wurde, wurden 1 ml des Reagenzes und 100 µl Probe 2 Stunden bei Raumtemperatur inkubiert. Anschließend wurde dreimal mit Leitungswasser gewaschen. Sodann wurde zur Nachweisreaktion 1 ml ABTS®-Subtratlösung zugegeben. Nach 1 Stunde wurde die Extinktion bei 405 nm photometrisch gemessen. Die Ergebnisse sind der Fig. 3 zu entnehmen.

### Beispiel 5

### T4-Test.

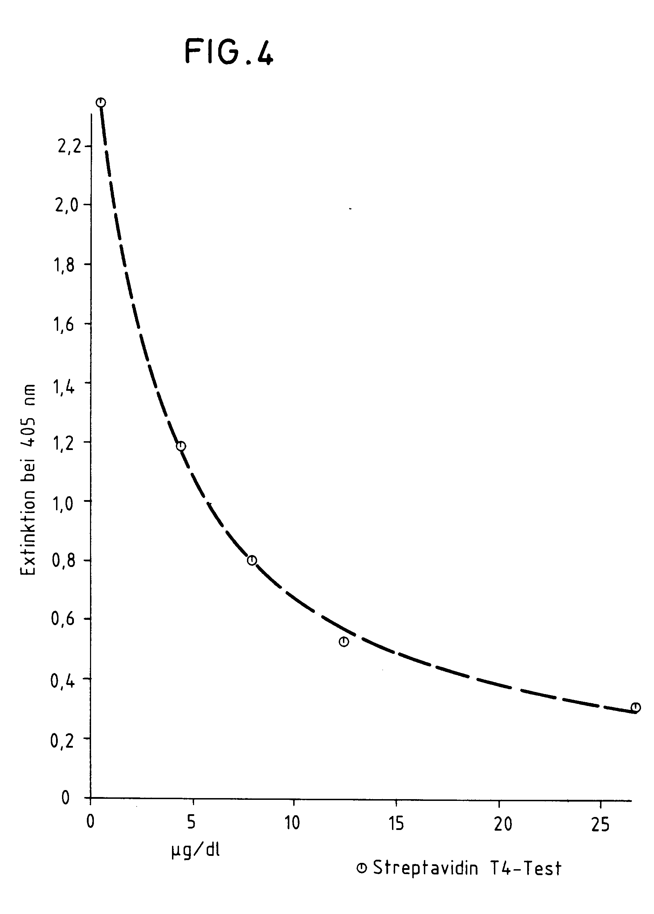

Es wurde T4 in einem kompetitiven Immunoassay unter Verwendung von biotinylierten Anti-T4-Antikörpern bestimmt. Dazu wurden in einem mit Streptavidin-Thermo-RSA-Konjugat beschichtetes Polystyroltube, wie es gemäß Beispiel 1 erhalten wurde, 500 µl eines Reagenzes, bestehend aus:
100 mU/ml Thyroxin-POD-Konjugat (hergestellt nach EP 209 155)
120 mmol/l Barbiturat,
18,2 mmol/l Phosphatpuffer pH: 8,6,
0,04 Gew.% ANS (8-Anilino-1-naphthalin-Sulfonsäure),
0,2 Gew.% Rinderserumalbumin
sowie 20 µl Probe 10 min bei Raumtemperatur inkubiert. Anschließend wurden 500 µl eines zweiten Reagenzes, bestehend aus:
1 µg/ml biotinyliertem polyklonalem Antikörper gegen T4,
120 mmol/l Phosphatpuffer pH: 8,6,
0,04 Gew.% ANS (8-Anilino-1-naphthalin-Sulfonsäure),
0,2 Gew.% Rinderserumalbumin,
zugegeben und weitere 30 min bei Raumtemperatur inkubiert. Nach dreimaligem Waschen mit Leitungswasser wurde dann 1 ml ABTS®-Substratlösung zugegeben und 30 min bei Raumtemperatur inkubiert. Anschließend wurde die Extinktion bei 405 nm photometrisch bestimmt. Die Ergebnisse sind der Fig. 4 zu entnehmen.

### Beispiel 6

### Anti HBₛ-Test

HBₛ-Antikörper werden in einem 1-Schritt-Sandwich-Immunoassay bestimmt. Zum Nachweis wird ein Reagenz mit der folgenden Zusammensetzung verwendet:
60 mU/ml eines Konjugats aus POD und HBₛ-Antigen
40 mmol/l Phosphatpuffer, pH 7,0
200 mmol/l Natriumtartrat
0,5 Gew.-% Pluronic F68
0,01 Gew.-% Phenol
0,2 % Rinderserumalbumin
0,1 Gew.-% Rinder-IgG
150 ng/ml biotinyliertes HBₛAg (hergestellt nach Immunol. Letters 8 (1984), 273).

In einem mit Streptavidin-Thermo-RSA-Konjugat beschichteten Polystyroltube gemäß Beispiel 1 wurde 1 ml dieses Reagenzes und 200 µl Probe 4 Stunden bei Raumtemperatur inkubiert. Anschließend wurde dreimal mit Leitungswasser gewaschen. Zur Nachweisreaktion wurde 1 ml ABTS-Substratlösung zugegeben. Nach 60 Minuten wurde die Extinktion bei 422 nm photometrisch gemessen.

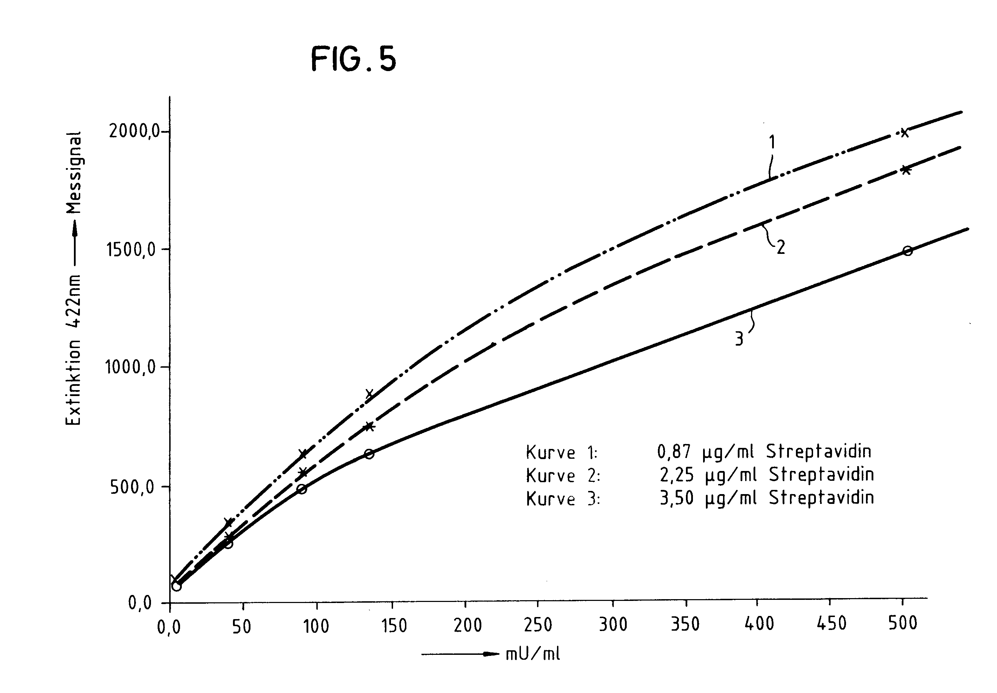

Der Anti-HBₛ-Test wurde, unter Verwendung von Tubes, mit unterschiedlicher Menge an immobilisiertem Streptavidin durchgeführt. Die Ergebnisse sind aus Fig. 5 zu entnehmen. Dabei zeigt sich, daß dann, wenn Streptavidin in Mengen eingesetzt wird, die über den erfindungsgemäß verwendeten Mengen liegen, der Test deutlich unempfindlicher wird.

### Beispiel 7

### Anti-HIV-Test

HIV-Antikörper werden in einem 2-Schritt-Sandwich-Immunoassay bestimmt. Zum Nachweis werden die Reagenzien mit folgender Zusammensetzung verwendet.

### Reagenz 1:

jeweils 10⁻⁷ mol/l eines oder mehrerer biotinylierter HIV-Antigene
40 mmol/l Phosphatpuffer, pH 7,0
0,9 Gew.-% Kochsalz
10 Vol.-% Rinderserum
Als Antigene wurden dabei folgende verwendet: gentechnologisch hergestellte HIV1-Antigene entsprechend HIV1-gp41 (gp41-rek., Centocor™-p121) und HIV1-p24 (p24-rek., Centocor™-pg2), chemisch synthetisierte Peptide aus HIV1-gp41 (gp41-pep, Wang et al., PNAS, 83, 6159, 1986) und HIV2-gp32 (gp32-pep, Gnann, J.W. et al., Science, 237, 1346, 1987). Diese Antigene wurden wie von Leary et al., PNAS, 80, 4045 (1983) mit Biotin markiert beschrieben.

### Reagenz 2:

20 mU/ml eines Konjugates aus Schafantikörper gegen Human-Immunglobulin und POD
40 mmol/l Phosphatpuffer, pH 7,0
0,05 Gew.-% Tween 20
0,2 % Rinderserumalbumin
0,2 % Rinder-IgG
In einem mit Streptavidin-Thermo-RSA-Konjugat beschichteten Polystyroltube gemäß Beispiel 1 wurden 1 ml des Reagenz 1 und 10 µl Humanserum oder -plasma 1 h bei Raumtemperatur inkubiert. Anschließend wurde 3 mal mit Leitungswasser gewaschen und 1 ml Reagenz 2 für 1 h bei Raumtemperatur inkubiert. Wieder wurde 3 mal mit Leitungswasser gewaschen und zur Nachweisreaktion 1 ml ABTS-Substratlösung zugegeben. Nach 60 min wurde die Extinktion bei 422 nm photometrisch gemessen.

Der Anti-HIV-Test wurde unter Verwendung einzelner HIV-Antigene und Antigenkombinationen durchgeführt. Dabei zeigte es sich, daß die Testführung im mit Streptavidin-Thermo-RSA-Konjugat beschichteten Polystyroltube sowohl für die Bestimmung einzelner Antigen-spezifischer Antikörper als auch für die simultane Bestimmung von mehreren Antikörpern oder Antikörperpopulationen geeignet ist (Screeningtest; Tabelle 1), gleichgültig, ob diese gegen den selben Virus oder mehrere Viren bzw. interessierende Antigene gerichtet sind.

**Tabelle 1**

| | Humanserum-Probe | | | | | | |
|---|---|---|---|---|---|---|---|
| HIV-Antigene | Neg. | Anti-HIV I | Anti-HIV I | Anti-HIV I | Anti-HIV II | Anti-HIV I | Anti-HIV II |
| HIV-Antigenmenge | | | | | | | |
| gp41-rek. | 52 | 1250 | 812 | 521 | 102 | 223 | 75 |
| p24-rek. | 43 | 786 | 1515 | 212 | 491 | 1531 | 263 |
| gp41-pep | 71 | 831 | 301 | 295 | 63 | 52 | 59 |
| gp32-pep | 38 | 41 | 50 | 62 | 1819 | 45 | 810 |
| gp41-, p24-rek. | 45 | 1402 | 1818 | 618 | 550 | 1559 | 266 |
| gp41-, p24-rek. + gp32-pep | 55 | 1380 | 1753 | 599 | 1723 | 1529 | 878 |

## Patentansprüche

1. Verfahren zur Bestimmung einer immunologisch nachweisbaren Substanz nach dem Prinzip des heterogenen Immunoassays unter Verwendung einer Festphase, an die eine der immunologisch aktiven Reaktionskomponenten gebunden ist,
**dadurch gekennzeichnet,** daß man als feste Phase ein Reaktionsgefäß verwendet, an dessen Innenoberfläche Streptavidin oder Avidin in einer solchen Menge gebunden ist, daß pro ml Reaktionsvolumen 0,1 bis 2,5 µg vorhanden sind.

2. Verfahren nach Anspruch 1, **dadurch gekennzeichnet,** daß man als Reaktionsgefäß eine Küvette verwendet, deren Wände an der Innenoberfläche mit Streptavidin oder Avidin beschichtet sind.

3. Verfahren nach einem der vorhergehenden Ansprüche, **dadurch gekennzeichnet,** daß man Streptavidin oder Avidin an ein lösliches Protein mit einem Molekulargewicht über etwa 500.000 kuppelt und dann das Konjugat aus Streptavidin oder Avidin und Protein an eine hydrophobe Festphase adsorbiert.

4. Verfahren nach einem der vorhergehenden Ansprüche, **dadurch gekennzeichnet,** daß man simultan mehrere Substanzen, insbesondere mehrere Antikörper bestimmt.

5. Reaktionsgefäß mit einander gegenüberliegenden optisch durchlässigen Wandbereichen und im übrigen zumindest teilweise im Bereich der zur Flüssigkeitsaufnahme vorgesehenen Innenwand mit Streptavidin oder Avidin beschichteten Wänden, wobei der für die Flüssigkeitsaufnahme bestimmte Innenraum des Behälters und der Streptavidin- bzw. Avidingehalt der Beschichtung so aufeinander abgestimmt sind, daß pro ml Reaktionsvolumen 0,1 bis 2,5 µg Streptavidin oder Avidin vorhanden sind.

6. Reaktionsgefäß nach Anspruch 5,
**dadurch gekennzeichnet,** daß das Streptavidin oder Avidin über ein lösliches Protein mit einem Molekulargewicht über 500.000 an die Festphase gebunden ist.

7. Reaktionsgefäß nach Anspruch 5 oder 6,
**dadurch gekennzeichnet,** daß das Reaktionsgefäß eine Küvette ist.

## Claims

1. Process for the determination of an immunologically detectable substance based on a heterogeneous immunoassay by use of a solid phase on which one of the immunologically active reaction components is bound,
**wherein**
a reaction vessel is used as the solid phase on the inner surface of which streptavidin or avidin is bound in such an amount that 0.1 to 2.5 µg are present per ml reaction volume.

2. Process as claimed in claim 1,
**wherein**
a cuvette is used as the reaction vessel whose walls are coated on the inner surface with streptavidin or avidin.

3. Process as claimed in one of the previous claims,
**wherein**
streptavidin or avidin is coupled to a soluble protein with a molecular weight above about 500 000 and then the conjugate of streptavidin or avidin and protein is adsorbed to a hydrophobic solid phase.

4. Process as claimed in one of the previous claims,
**wherein**
several substances in particular several antibodies are determined simultaneously.

5. Reaction vessel with optically transparent wall areas which face one another and with avidin or streptavidin coated walls which are at least partially within the inner wall region intended as a receptacle for liquid, wherein the inner space of the container intended as a receptacle for liquid and the respective streptavidin or avidin content of the coating are so matched that 0.1 to 2.5 µg streptavidin or avidin are present per ml reaction volume.

6. Reaction vessel as claimed in claim 5,
**wherein**
streptavidin or avidin is bound to the solid phase via a soluble protein with a molecular weight above 500 000.

7. Reaction vessel as claimed in claim 5 or 6,
**wherein**
the reaction vessel is a cuvette.

## Revendications

1. Procédé de détermination d'une substance décelable immunologiquement selon le principe de l'immuno-analyse hétérogène au moyen d'une phase solide à laquelle est lié l'un des composants réactionnels immunologiquement actifs, caractérisé en ce que l'on utilise comme phase solide un récipient réactionnel à la surface interne duquel est liée de la streptavidine ou de l'avidine en une quantité telle qu'il y en a 0,1 à 2,5 µg par ml de volume réactionnel.

2. Procédé selon la revendication 1, caractérisé en ce que l'on utilise comme récipient réactionnel une cuvette dont les parois sont recouvertes de streptavidine ou d'avidine au niveau de la surface interne.

3. Procédé selon l'une des revendications précédentes, caractérisé en ce que l'on couple la streptavidine ou l'avidine à une protéine soluble d'une masse moléculaire supérieure à 500000 environ puis l'on adsorbe le conjugué de streptavidine ou d'avidine et de protéine sur une phase solide hydrophobe.

4. Procédé selon l'une des revendications précédentes, caractérisé en ce que l'on détermine simultanément plusieurs substances, en particulier plusieurs anticorps.

5. Récipient réactionnel comportant des domaines de parois optiquement transparents mutuellement opposés et en outre des parois recouvertes de streptavidine ou d'avidine au moins en partie dans le domaine de la paroi interne prévue pour la réception du liquide, l'espace interne du récipient qui est destiné à la réception du liquide et la teneur en streptavidine ou en avidine du revêtement étant ajustés l'un par rapport à l'autre de telle manière qu'il y a par ml de volume réactionnel 0,1 à 2,5 µg de streptavidine ou d'avidine.

6. Récipient réactionnel selon la revendication 5, caractérisé en ce que la streptavidine ou l'avidine est liée à la phase solide par l'intermédiaire d'une protéine soluble de masse moléculaire supérieure à 500000.

7. Récipient réactionnel selon la revendication 5 ou 6, caractérisé en ce que le récipient réactionnel est une cuvette.
